# EUROPÄISCHE PATENTSCHRIFT

(11) **EP 4 271 454 B1**
(45) Veröffentlichungstag und Bekanntmachung des Hinweises auf die Patenterteilung: **12.02.2025**
(21) Anmeldenummer: 21847702.4
(22) Anmeldetag: 28.12.2021
(51) Int. Cl.: A61M 16/04

(54) **BEATMUNGSVORRICHTUNG MIT STOMA-MANSCHETTE**
VENTILATION DEVICE WITH STOMA CUFF
DISPOSITIF DE VENTILATION AVEC MANCHON DE STOMIE

(30) Priorität: 30.12.2020 DE 102020135146
(43) Veröffentlichungstag der Anmeldung: 08.11.2023
(73) Patentinhaber: Coloplast A/S, 3050 Humlebaek (DK)
(72) Erfinder: SCHNELL, Ralf, 63500 Seligenstadt (DE)
(86) Internationale Anmeldenummer: PCT/EP2021/087724
(87) Internationale Veröffentlichungsnummer: WO 2022/144357

(56) Entgegenhaltungen:
- DE-B3- 10 361 428
- US-A- 5 499 625

## Beschreibung

Die vorliegende Erfindung betrifft eine Beatmungsvorrichtung, insbesondere eine Tracheostomiekanüle, mit einem Kanülenrohr, einer ersten aufblasbaren Manschette zur Abdichtung der Trachea und einer zweiten aufblasbaren Manschette zur Abdichtung des Stomas.

Um sicherzustellen, dass die Atemluft sowohl beim Ein- als auch beim Ausatmen nahezu vollständig durch eine Tracheostomiekanüle strömt, ist bei vielen handelsüblichen Produkten dieser Art eine aufblasbare Manschette, der sogenannte Cuff, vorgesehen. Ein solcher Cuff dichtet die Luftröhre um die Tracheostomiekanüle herum ab, damit keine Luft über die natürlichen Atemwege strömen kann. Da auf diese Weise auch keine Ausatemluft an den stimmbildenden Organen vorbeigeführt werden kann, hat ein Patient mit geblocktem Cuff typischerweise keine Möglichkeit zur Stimmerzeugung (Phonation).

Für diese Zwecke wurde die Methode der sogenannten Above-Cuff-Vocalization (ACV) entwickelt, bei der mithilfe einer separaten Luftleitung von außen Luft in den zwischen dem Cuff und der Stimmritze liegenden subglottischen Raum eingebracht wird. Diese Luft kann dann über die natürlichen Atemwege entweichen und vom Patienten mit etwas Übung zum Sprechen verwendet werden.

Bei einigen handelsüblichen Tracheostomiekanülen ist für die Luftzuführung im subglottischen Bereich eine Sprechluft-Leitung in Form eines auf dem Kanülenrohr befestigten Schlauches vorgesehen. Das äußere Ende des Schlauches kann mit einer Druckluftquelle verbunden werden, z.B. über ein Y-Stück oder einen Konnektor. Mit einem Ventil, einem Regler oder einem Fingertip-Konnektor kann gesteuert werden, wann die Druckluft über die Sprechluft-Leitung in den subglottischen Bereich einströmt.

Anstelle von speziell für ACV hergestellten Tracheostomiekanülen werden oft Kanülen verwendet, die einen subglottischen Absaugkanal aufweisen. Der subglottische Absaugkanal dient eigentlich dazu, Sekret, das sich über dem Cuff angesammelt hat, zu entfernen. Hierfür wird an dem Absaugkanal ein Unterdruck angelegt, mit dem das Sekret aus dem subglottischen Bereich abgesaugt werden kann. Ein Beispiel für eine Tracheostomiekanüle mit subglottischer Absaugung wird in EP 1 219 317 B1 beschrieben.

Dreht man bei einer Kanüle mit subglottischem Absaugkanal die Strömungsrichtung nach dem Absaugen um, kann über den Absaugkanal Druckluft in den subglottischen Bereich eingeleitet werden, die dann als Sprechluft über die natürlichen Atemwege entweichen kann. Die Sprechluftzuführung über einen subglottischen Absaugkanal ist allerdings mit dem Nachteil behaftet, dass nach dem Absaugen noch an der Kanalwand befindliches Sekret durch die Druckluft eintrocknet. Das eingetrocknete Sekret klebt an der Kanalwand fest und verhärtet diese. Mit der Zeit führt dies dazu, dass der Absaugkanal undurchlässig wird und somit seine Funktion verliert. Daher ist eine separate Sprechluftzuführung erstrebenswert, die sich insbesondere auch bei solchen Kanülen bzw. Tuben gut realisieren lässt, die bereits einen subglottischen Absaugkanal aufweisen.

Alle bisher genannten Lösungswege funktionieren nur, wenn die Tracheostomiekanüle eng im Tracheostoma anliegt. Sehr viele Patienten haben jedoch ein Tracheostoma (im folgenden Stoma genannt), das deutlich größer ist als der Außendurchmesser der Tracheostomiekanüle. Insbesondere bei chirurgisch angelegten Stomata oder auch bei älteren Stomata ist dies häufig der Fall.

Somit gibt es bei diesen Patienten eine Lücke zwischen Kanüle und dem das Stoma umgebende Gewebe. Da die Luft stets den Weg des geringsten Widerstands nimmt, entweicht in diesem Fall ein Großteil der Luft durch diese Öffnung und nicht durch die natürlichen Atemwege. Dies hat einen ausgesprochen negativen Einfluss auf die Sprachqualität.

Prinzipiell besteht die Möglichkeit ein zu großes Stoma mit Hilfe einer speziell für den Patienten angefertigte Epithese abzudichten. Da dies jedoch sehr aufwändig ist, wird solch eine Epithese leider nicht für jeden Patienten angefertigt, der davon profitieren würde.

In US 4,459,984 A1 wird eine Tracheostomiekanüle beschrieben, die an der Tracheostomiekanüle auf Höhe des Stomas eine aufblasbare Manschette aufweist, die das Stoma abdichten soll. Es hat sich jedoch gezeigt, dass durch solch eine Manschette leicht Druckstellen entstehen können, die für den Patienten sehr schmerzhaft sind. Der dauerhafte Druck auf das Gewebe scheint für das empfindliche Stoma sehr problematisch zu sein.

Es wurde daher versucht, mit Manschetten zu arbeiten, die aus besonders weichem Silikon bestehen. Wenn man die Manschette aus einem solchen weichen Silikon ausführt, tritt allerdings das Problem auf, dass die Luft mit der Zeit aus der Silikonmanschette durchdiffundiert. Somit wird der Durchmesser der gefüllten Manschette mit der Zeit kleiner. Dies führt oft dazu, dass der Anwender die Manschette mit mehr Luft als eigentlich nötig befüllt, damit die Abdichtung möglichst lange andauert. Dies begünstigt dann allerdings wiederum die Ausbildung von Druckstellen.

Im Übrigen ist der Durchmesser des Stomas nicht immer konstant, sondern kann sich durch die Körperhaltung des Patienten und auch durch den Muskeltonus des Patienten verändern. Dies hat dann direkten Einfluss darauf, mit welchem Druck die Manschette auf das Stomagewebe einwirkt. Wenn der Druck dauerhaft höher ist als 30 mbar bekommt der Patient Druckstellen. Wenn der Druck dagegen zu gering ist, treten Leckagen auf, die wiederum die Phonation erschweren.

Nachfolgende Dokumente sind auch aus dem Stand der Technik bekannt, nämlich DE 103 61 428 B3 und US 5 499 625 A.

Um die Nachteile, die mit den im Stand der Technik bereits existierenden technischen Lösungen verbunden sind, zu überwinden, wird erfindungsgemäß eine Beatmungsvorrichtung, insbesondere eine Tracheostomiekanüle, vorgeschlagen mit
▪ einem Kanülenrohr, dessen Rohrwand einen Extrakorporalabschnitt, einen Stomaabschnitt und einen Trachealabschnitt aufweist,
▪ einer ersten aufblasbaren Manschette, die sich im Trachealabschnitt an der Außenseite der Rohrwand ringförmig um das Kanülenrohr erstreckt,
▪ einer ersten Luft-Leitung, die einen Luftauslass in das Lumen der ersten Manschette aufweist,
▪ einer zweiten aufblasbaren Manschette, die sich im Stomabschnitt an der Außenseite der Rohrwand ringförmig um das Kanülenrohr erstreckt, und
▪ einer zweiten Luft-Leitung, die einen Luftauslass in dem Bereich zwischen Stomaabschnitt und der ersten Manschette aufweist,
dadurch gekennzeichnet, dass die zweite Luft-Leitung im Bereich des Stomaabschnitts einen zusätzlichen Luftauslass in das Lumen der zweiten Manschette aufweist.

Die erfindungsgemäße Beatmungsvorrichtung kommt bei bestimmungsgemäßem Gebrauch im Bereich des Stomaabschnitts an dem das Stoma eines Patienten umgebenden Gewebe zum anliegen. Ausgehend von diesem Stomaabschnitt erstrecken sich der Extrakorporalabschnitt in proximaler Richtung und der Trachealabschnitt in distaler Richtung.

Die Begriffe "distal" und "proximal" werden im Sinne der vorliegenden Erfindung aus der Sicht eines die Beatmungsvorrichtung anwendenden Arztes verwendet, d.h. das proximale Ende ist das Ende der Beatmungsvorrichtung, das nach dem Einführen in die Trachea außerhalb des Patientenkörpers verbleibt, während das distale Ende in die Trachea des Patienten eingeführt wird.

Der Begriff "Leitung" wird im Zusammenhang mit der vorliegenden Erfindung im Sinne einer luftführenden Leitung verstanden, in die an dem wenigstens einen vorgesehenen Lufteinlass Luft einströmen kann und aus der die Luft an dem wenigstens einen vorgesehenen Luftauslass wieder ausströmen kann, nachdem die Luft durch die ansonsten luftdichte Leitung über eine bestimmte Strecke hindurchgeströmt ist.

Durch die erfindungsgemäß vorgeschlagene zweite Luft-Leitung, die im Bereich des Stomaabschnitts einen zusätzlichen Luftauslass in das Lumen der zweiten Manschette aufweist, wird sichergestellt, dass die zweite Manschette dann, wenn die zweite Luft-Leitung von Sprechluft durchströmt wird, aufgeblasen wird und so einen möglicherweise zwischen Tracheostomiekanüle und Stomagewebe bestehenden Spalt im Moment der Sprechluftzuführung abdichtet. Hierdurch wird die Qualität der Stimmbildung deutlich verbessert, ohne dass die Gefahr besteht, dass am Stoma Druckstellen auftreten, weil die Druckbelastung des Stomagewebes durch die aufgeblasene Manschette nur im Moment des ACV-Prozesses vorliegt nicht aber während der Sprechpausen, bei denen keine Luft durch die Sprechluft-Leitung strömt.

Vorzugweise nimmt die zweite Manschette bei einem Sprechluft-Volumenstrom von 2 bis 10 Litern/Minute im Stoma ein Volumen ein, mit dem das Stoma abgedichtet wird. Bei besonders bevorzugten Ausführungsformen wird das abdichtende Volumen von der zweiten Manschette im Bereich von 4 bis 6 Litern/Minute eingenommen. Der für das Abdichten erforderliche Durchmesser der zweiten Manschette bei einem Sprechluft-Volumenstrom von 2 bis 10 Litern/Minute bzw. im Bereich von 4 bis 6 Litern/Minute hängt von der Größe des Stomas des Patienten ab, an das sich die Manschette während der Sprechphase anschmiegt.

Der Begriff "Umgebungsdruck" bezeichnet im Sinne der vorliegenden Erfindung den auf die Manschette wirkenden Druck. Bei einer nicht im Gebrauch befindlichen Beatmungsvorrichtung, die nicht in ein Stoma eingesetzt vorliegt und durch deren zweite Luft-Leitung keine Sprechluft geführt wird, entspricht der Umgebungsdruck dem Atmosphärendruck und wirkt gleichermaßen auf die nach außen und die nach innen gewandte Seite von zweiter Luft-Leitung und zweiter Manschette.

Wird Sprechluft durch die zweite Luft-Leitung (Sprechluft-Leitung) geführt, wirkt auf die nach innen gewandte Seite der Manschette ein entsprechend erhöhter Überdruck. Erhöht sich dann auch der von außen auf die Manschette wirkende Umgebungsdruck, wirkt dieser Überdruck dem durch die Sprechluft auf die nach innen gewandte Seite der Manschette wirkenden erhöhten Druck entgegen.

Der von außen auf die Manschette wirkende Umgebungsdruck kann entweder durch umliegendes Gewebe auf die Manschette wirkender Druck sein oder von außen auf die Manschette der Luftleitung wirkender Luftdruck.

Bei bestimmten Ausführungsformen der Erfindung reduziert sich der maximale Durchmesser der zweiten Manschette (Stoma-Manschette) bei einem auf die Außenseite der Manschette wirkenden Überdruck von über 30 mbar um wenigstens 10% im Vergleich zum maximalen Durchmesser, der vorliegt, wenn durch die zweite Luft-Leitung Sprechluft mit einem Sprechluft-Volumenstrom von 5 Litern pro Minute strömt. Strömt keine Sprechluft mehr durch die zweite Luft-Leitung, verringert sich der Durchmesser bereits bei einem auf die Außenseite der Manschette wirkenden Überdruck von weniger als 30 mbar um wenigstens 10%.

Bei bestimmten Ausführungsformen ist die zweite Luft-Leitung eine reine Sprechluft-Leitung, durch die nur Sprechluft geführt wird. Bei anderen Ausführungsformen ist die zweite Luft-Leitung eine subglottische Absaug-Leitung, durch die in die eine Richtung subglottisches Sekret abgesaugt werden kann und durch die in die andere Richtung Sprechluft geführt werden kann.

Bei verschiedenen Ausführungsformen der Erfindung verläuft die zweite Luft-Leitung entweder in der Rohrwand des Kanülenrohrs oder auf der Außenseite der Rohrwand des Kanülenrohrs oder an der Innenseite der Rohrwand des Kanülenrohrs.

Bei bestimmten Ausführungsformen endet die zweite Luft-Leitung in der Richtung proximaldistal am oder unmittelbar nach dem Übergang vom Stomaabschnitt in den Trachealabschnitt und mündet mit Ihrem Luftauslass dort in die Trachea.

Bei anderen Ausführungsformen erstreckt sich die zweite Luft-Leitung im Anschluss an den Stomaabschnitt in distale Richtung über bis zu 20 %, bis zu 40 %, bis zu 60 % oder bis zu 80 % der Länge der Strecke zwischen dem Übergang vom Stomaabschnitt in den Trachealabschnitt und der ersten Manschette (Trachea-Manschette).

Die Sprechluft wird typischerweise durch eine Drucklufteinrichtung bereitgestellt, die durch das Lumen der zweiten Luft-Leitung einen Sprechluft-Volumenstrom von wenigstens 5 l/min bereitstellen kann. Bei dem Sprechluftvolumenstrom handelt es sich um das Volumen, das die zweite Luft-Leitung bei bestimmungsgemäßer Anwendung der erfindungsgemäßen Beatmungsvorrichtung innerhalb einer bestimmten Zeit durchströmt. Vorzugsweise handelt es sich hierbei um das Normvolumen bei standardisierter Referenzatmosphäre bei 20 °C und 1000 mbar. Vorzugsweise wird der Sprechluft-Volumenstrom bei trockener Druckluft gemessen (p(H₂O) = 0 kPA). Vorzugsweise wird das Normvolumen bei standardisierter Referenzatmosphäre gemäß DIN 1945-1 bestimmt.

Bei manchen Ausführungsformen wird der maximale Durchmesser der Stoma-Manschette durch eine Dehnung des Manschettenmaterials erreicht, die sich bei einem Sprechluft-Volumenstrom von 5 l/min und einem Umgebungsdruck von 1000 mbar einstellt. Die Reduzierung des Durchmessers durch erhöhten Umgebungsdruck bzw. durch verringerten Volumenstrom stellt sich bei diesen Ausführungsformen dadurch ein, dass die Dehnung des Manschettenmaterials wieder zurückgeht.

Bei anderen Ausführungsformen wird der maximale Durchmesser dadurch erreicht, dass bei einem Sprechluft-Volumenstrom von 5 l/min und einem Umgebungsdruck von 1000 mbar das Manschettenmaterial durch die Sprechluft, je nach Ausführungsform entgegen der Schwerkraftwirkung bzw. der Eigenspannung der Manschette, aufgespannt wird, ohne dass hierbei ein Dehnung der Manschette erfolgt. Die Reduzierung des Durchmessers durch erhöhten Umgebungsdruck bzw. durch verringerten Volumenstrom stellt sich bei diesen Ausführungsformen dadurch ein, dass die Manschette wenigstens teilweise zusammenfällt. Bei speziellen Ausführungsformen wird der Durchmesser teilweise durch eine Dehnung der Manschette und teilweise durch Aufspannen der Manschette durch ein Sprechluft-Volumenstrom von 5 l/min bei einem Umgebungsdruck von 1000 mbar erreicht.

Manche Ausführungsformen der Erfindung sind dadurch gekennzeichnet, dass die zweite aufblasbare Manschette bei einem durch die zweite Luftleitung durchgeleiteten Luftstrom von 5 Litern pro Minute im freien Zustand außerhalb eines Patienten einen Außendurchmesser aufweist, der um wenigstens 20% größer ist als der Außendurchmesser des Kanülenrohres auf Höhe der Manschette. Dies gilt insbesondere bei Ausführungsformen der Erfindung, bei denen sich das Manschettenmaterial nicht wesentlich ausdehnt, sondern sich durch Faltenwurf an die Stomawand anschmiegt (HVLP-Cuff = *High Volume Pressure Low Cuff*)*.* Vorzugweise ist der Außendurchmesser der Manschette im voll aufgeblasenen freien Zustand hier sogar wenigstens 30 %, wenigstens 40 % oder wenigstens 50 % größer sein als der Außendurchmesser des Kanülenrohrs.

Bei einem vorgegebenen Luftstrom von 5 Litern/Minute ist das System mit HVLP-Manschette vorzugsweise derart aufeinander abgestimmt, dass sich in der Manschette ein Druck zwischen 10 mbar und 60 mbar, bevorzugt 20 bis 30 mbar, ausbildet. Bei einem System mit dehnbarer Manschette ist dagegen ein Druck zwischen 40 und 80 mbar bevorzugt, da ein Teil dieses Drucks erforderlich ist, um die Rückstellkräfte des elastischen Materials zu überwinden.

Bei bestimmten Ausführungsformen der vorliegenden Erfindung ist die Sprechluftzuführung zumindest abschnittsweise von einer Lage einer Folie gebildet, die auf der Außenseite der Rohrwand des Kanülenrohrs befestigt ist. Bei einer alternativen Ausführungsform wird die Sprechluftzuführung zumindest abschnittsweise von einem aus der Folie bestehenden Schlauch gebildet, der auf der Außenseite der Rohrwand des Kanülenrohrs befestigt ist. Während bei der zuerst genannten Alternative das Lumen der zweiten Luft-Leitung auf der einen Seite von der Lage der Folie und auf der anderen Seite von der Außenseite der Rohrwand des Kanülenrohrs definiert wird, wird bei der zuletzt genannten Alternative das Lumen der Sprechluftzuführung ausschließlich von dem aus der Folie bestehenden Schlauch definiert.

Bei bestimmten Ausführungsformen weisen die Folie oder der Schlauch eine vorgeformte Geometrie mit geringerem Querschnitt auf, wobei sich der vollständige Querschnitt erst dann ausbildet, wenn die Sprechluft das Lumen durchströmt. Ohne Sprechluftstrom nimmt die Folie oder der Schlauch wieder den Querschnitt der vorgeformten Geometrie ein. Die vorgeformte Geometrie kann beispielsweise eine oder mehrere in Längsrichtung verlaufende Einbuchtungen vorgeben, beispielweise durch unterschiedliche Wandstärken der Folie.

Die Folienlage bzw. der Folienschlauch werden bei bestimmten Ausführungsformen auf der Außenseite der Rohrwand des Kanülenrohrs durch Kleben oder Schweißen befestigt.

Die Aufbringung einer Lage der Folie auf die Außenseite der Rohrwand des Kanülenrohrs ist mit dem Vorteil verbunden, dass relativ wenig Folienmaterial verwendet werden muss. Zum anderen ergibt sich hierdurch eine typischerweise halbmondförmige Gestalt des Querschnitts des Lumens der zweiten Luft-Leitung, wodurch ein Lumen erreicht werden kann, das in radialer Richtung relativ wenig aufträgt.

Die Anbringung der zweiten Luft-Leitung in Form eines aus der Folie bestehenden Schlauches hat wiederum den Vorteil, dass ein solcher Schlauch an sich eine große Zuverlässigkeit im Hinblick auf die Dichtigkeit mit sich bringt, während im Falle der Anbringung einer Lage der Folie auf der Außenseite der Kanülenwand die Verbindung luftdicht erfolgen muss, um die erforderliche Dichtigkeit zu erreichen.

Bei handelsüblichen Kanälen für eine subglottische Absaugung wäre solch eine flexible Ausführung, wie sie hier beschrieben wird nicht zielführend, da ein weicher Absaugkanal beim Anlegen des für die Absaugung benötigten Vakuums kollabieren würde. Da eine effektive Absaugung im kollabierten Zustand nicht möglich wäre, müssen die Kanäle für die subglottische Absaugung zwangsläufig recht unflexibel ausgeführt sein. Dies führt in vielen Fällen zu Druckstellen.

Bei bestimmten Ausführungsformen ist die Geometrie der zweiten Luft-Leitung so gewählt, dass bei einem Sprechluftvolumenstrom von 5 l/min der hydraulische Durchmesser der zweiten Luft-Leitung bei einem Umgebungsdruck von 1000 mbar mindestens 2,5 mm beträgt.

Der hydraulische Durchmesser des Luftaustritts sollte so dimensioniert sein, dass sich einerseits bei einem Luftstrom von 5 l/min durch diese Öffnung in der Manschette ein ausreichender Druck aufbaut, damit sich die Manschette derart an die Stomawand anschmiegt, dass eine ausreichende Abdichtung entsteht. Andererseits sollte der Druck nicht so groß sein, dass diese Abdichtung für den Patienten als schmerzhaft empfunden wird. Vorzugweise sollte der Anpressdruck auf das Gewebe nicht größer sein als 40 mbar. Da der maximale Druck lediglich für die kurze Zeit der Sprechluftzuführung stattfindet ist das Risiko von Druckstellen durch die zweite Luft-Leitung nahezu ausgeschlossen. Je nach Ausführung liegt der Querschnitt des Luftaustritts im Bereich von 1 bis 4,5 mm².

Bei manchen Ausführungsformen weist das Material der zweiten Manschette eine besonders hohe Reißfestigkeit auf und ist wenigstens bis zu einem Sprechluftdruck von 200 mbar reißfest. Noch bevorzugter ist die zweite Manschette bis zu einem Sprechluftdruck von 500 mbar reißfest.

Die Reißfestigkeit und auch die Variabilität des Lumens der zweiten Manschette werden zum Teil durch die Schichtdicke (Wandstärke) des Manschettenmaterials beeinflusst und teilweise durch die Eigenschaften des Materials, aus dem die Manschette besteht. Bei bestimmten Ausführungsformen der Erfindung besteht das Manschettenmaterial aus einer Folie mit einer Wandstärke im Bereich von 5 bis 300 µm. Bei manchen Ausführungsformen beträgt die Wandstärke des Materials der zweiten Manschette wenigstens 10 µm, wenigstens 20 µm oder wenigstens 30 µm. Vorzugsweise beträgt die Wandstärke bei bestimmten Ausführungsformen höchstens 250 µm oder höchstens 200 µm.

Vorzugsweise hat das Material der zweiten Manschette bei bestimmten Ausführungsformen eine Bruchdehnung (nach DIN EN ISO 527) im Bereich von 150-600%.

Bei bestimmten Ausführungsformen besteht die zweite aufblasbare Manschette aus einem thermoplastischen Kunststoffpolymer mit einem Shore A im Bereich von 5 bis 90.

Bei bestimmten Ausführungsformen besteht die zweite aufblasbare Manschette aus einem thermoplastischen Kunststoffpolymer, das unter PVC oder Polyurethan ausgewählt ist. Weitere Kunststoffpolymere als Material für die zweite aufblasbare Manschette sind PE, PP, EVA, PEBAX und Silikon.

Es kann ohne Probleme als Manschettenmaterial auch ein Polymer eingesetzt werden, das wie Silikon eine recht hohe Permeabilität für Luft aufweist, da sich der Luftdruck in jeder Sprechphase neu ausbildet.

Bei bestimmten Ausführungsformen weist die erfindungsgemäße Beatmungsvorrichtung zusätzlich eine separate Absaugleitung zum Absaugen von subglottischem Sekret auf.

Bei den Ausführungsformen, bei denen eine separate Absaugleitung zum Absaugen von subglottischem Sekret vorgesehen ist, kann diese entweder in der Wand der Tracheostomiekanüle eingebracht sein oder auf der Außenseite der Rohrwand befestigt sein. Im letzteren Fall verläuft die Folie der zweiten Luft-Leitung auf der Oberseite der Absaugleitung seitlich neben der Absaugleitung. Bei einer alternativen Ausführungsform überspannt die Folie der zweiten Luft-Leitung die Absaugleitung in Längsrichtung wenigstens abschnittsweise.

Vorzugsweise ist der Luftauslass der zweiten Luft-Leitung in proximaler Richtung in einer Entfernung von wenigstens 2 mm vom Sekreteinlass der Absaugleitung angeordnet. Dieser Mindestabstand gewährleistet, dass die Luft zum Sprechen nicht durch eine Restmenge an verbliebenem Sekret strömen muss. Dies würde die Sprachqualität verschlechtern und hätte zudem auch hygienische Nachteile, da sich Aerosole bilden könnten.

Bei bestimmten Ausführungsformen der Erfindung ist an dem proximalen Ende der zweiten Luft-Leitung ein Verbindungsstück für den Anschluss an eine Druckluftquelle vorgesehen, wie z.B. ein Y-Stück oder ein Konnektor. Bei bestimmten Ausführungsformen weist das Verbindungsstück ein Ventil, einen Regler und/oder eine mit einem Finger verschließbare Öffnung auf (=Fingertip-Konnektor). Hierüber kann gesteuert werden, wann und ggf. auch wieviel Druckluft über die zweite Luft-Leitung in den subglottischen Bereich einströmt.

Bestimmte Ausführungsformen der Erfindung sind dadurch gekennzeichnet, dass die zweite Luft-Leitung im Bereich des Extrakorporalabschnitts aus einem flexiblen Kunststoffschlauch besteht, der eine größere Härte und/oder Wandstärke aufweist, als die Folie, aus der die Sprechluftzuführung zumindest abschnittsweise im Stomaabschnitt und/oder Trachealabschnitt besteht.

Bei bestimmten Ausführungsformen der Erfindung weist die zweite Luft-Leitung am Luftauslass an deren distalem Ende zusätzlich noch ein Ventil auf. Erfindungsgemäß sollte dieses Ventil so eingerichtet sein, dass es den Luftaustritt in den subglottischen Bereich erlaubt, während es den Lufteintritt aus dem subglottischen Bereich in die zweite Luft-Leitung verhindert. Die Ventilfunktion ermöglicht es, Unterdruck in der zweiten Luft-Leitung zu erzeugen, um erforderlichenfalls den Querschnitt des Lumens der zweiten Luft-Leitung durch Anlegen von Unterdruck gezielt zu reduzieren. Die im Stoma befindliche Manschette schmiegt sich dann an das Kanülenrohr der Tracheostomiekanüle an.

Bei bestimmten Ausführungsformen wird die Ventilfunktion durch ein Klappenventil bereitgestellt. Bei alternativen Ausführungsform wird die Ventilfunktion dadurch bereitgestellt, dass der letzte Abschnitt des Luftaustritts im Vergleich zu den übrigen Abschnitten der zweiten Luft-Leitung deutlich dünnwandiger ausgestaltet ist, sodass sich dieser Abschnitt bei Anlegen eines Unterdruckes verschließt. Vorzugsweise beträgt die Länge des besonders dünnwandig ausgestalteten Abschnitts des Luftaustritts 0,3-1 cm oder mehr.

Für Zwecke der ursprünglichen Offenbarung wird darauf hingewiesen, dass sämtliche Merkmale, wie sie sich aus der vorliegenden Beschreibung, den anhängenden Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

In den anhängenden Figuren und der dazugehörigen Figurenbeschreibung sind einzelne konkrete Ausführungsformen der Erfindung schematisch dargestellt. Diese konkreten Ausführungsformen sind lediglich ein Beispiel für mögliche Merkmalskombinationen. Die Erfindung ist jedoch keinesfalls auf diese konkreten Ausführungsformen beschränkt, sondern umfasst alle vom Schutzumfang der Patentansprüche abgedeckten Ausführungsformen, auch wenn sie nicht explizit dargestellt oder beschrieben sind.

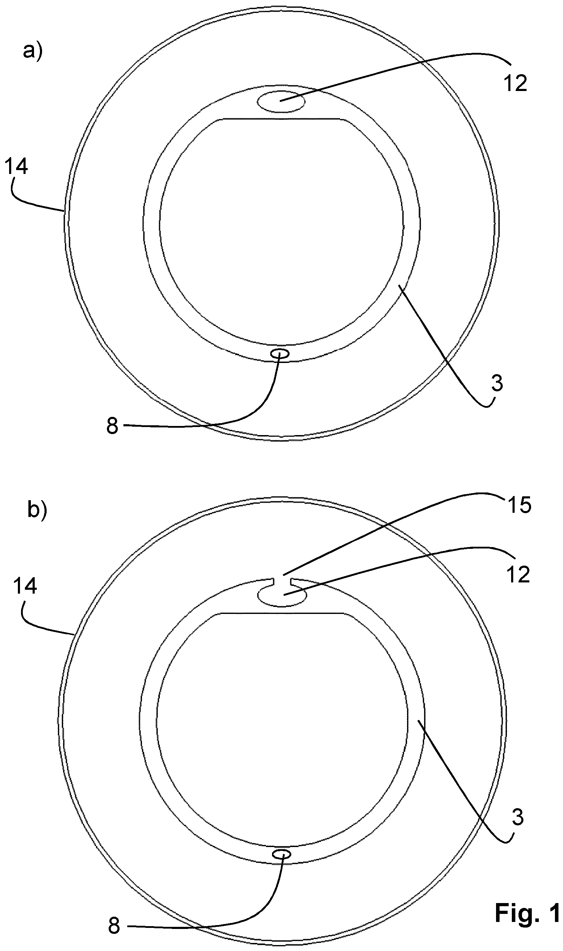

- Figur 1:: Die Abbildungen a) und b) von Figur 1 stellen schematische Querschnitte durch das Kanülenrohr im Stomaabschnitt einer Ausführungsform einer erfindungsgemäßen Beatmungsvorrichtung dar, bei der die Sprechluftleitung in Form eines in der Rohrwand des Kanülenrohres verlaufenden Kanals realisiert ist, wobei sich der Querschnitt in der Abbildung b) auf der Höhe eines zusätzlichen Luftauslasses in das Lumen der ersten Manschette befindet.

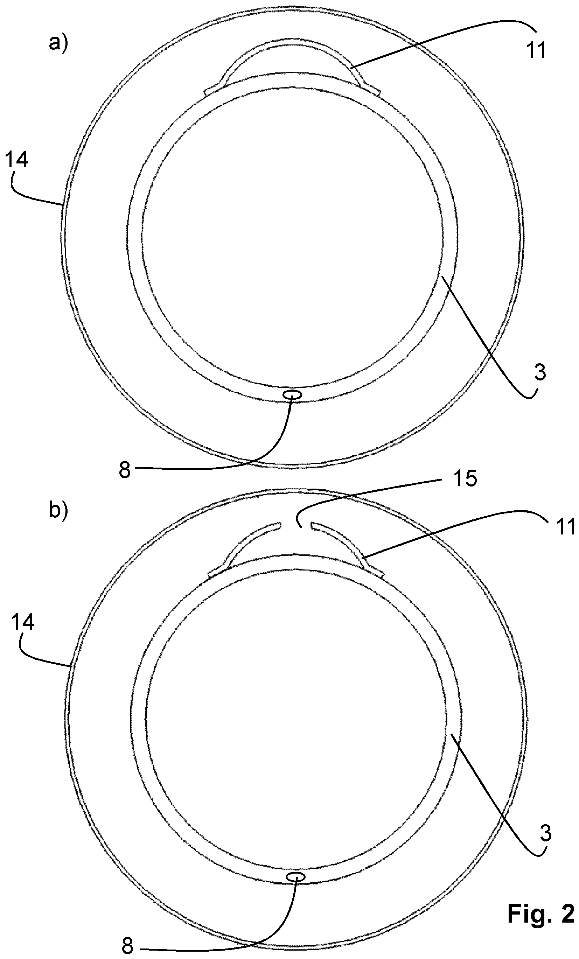

- Figur 2:: Die Abbildungen a) und b) von Figur 1 stellen schematische Querschnitte durch das Kanülenrohr im Stomaabschnitt einer Ausführungsform einer erfindungsgemäßen Beatmungsvorrichtung dar, bei der die Sprechluftleitung in Form einer auf der Außenseite des Kanülenrohres angeordneten Folienlage realisiert ist, wobei sich der Querschnitt in der Abbildung b) auf der Höhe eines zusätzlichen Luftauslasses in das Lumen der ersten Manschette befindet.

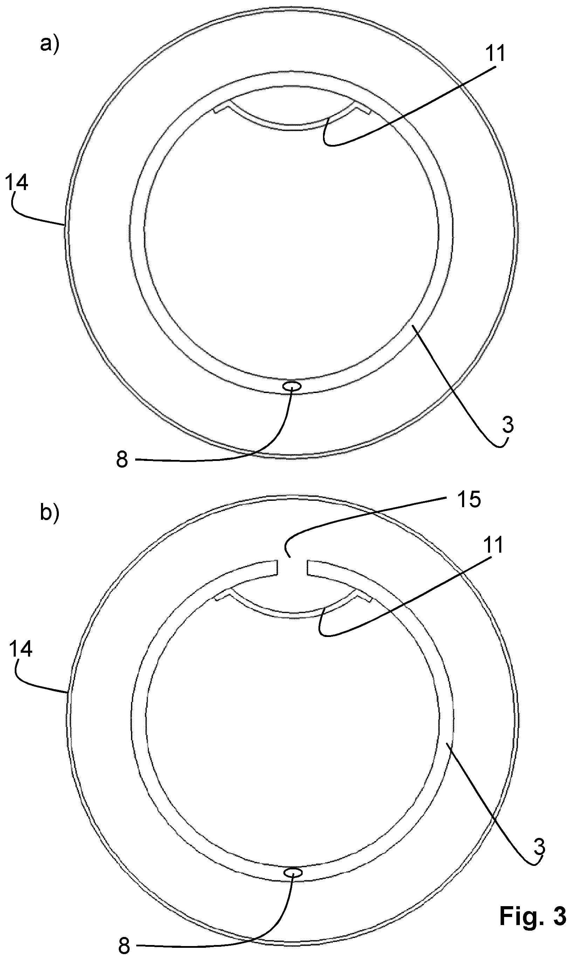

- Figur 3:: Die Abbildungen a) und b) von Figur 1 stellen schematische Querschnitte durch das Kanülenrohr im Stomaabschnitt einer Ausführungsform einer erfindungsgemäßen Beatmungsvorrichtung dar, bei der die Sprechluftleitung in Form einer auf der Innenseite des Kanülenrohres angeordneten Folienlage realisiert ist, wobei sich der Querschnitt in der Abbildung b) auf der Höhe eines zusätzlichen Luftauslasses in das Lumen der ersten Manschette befindet.

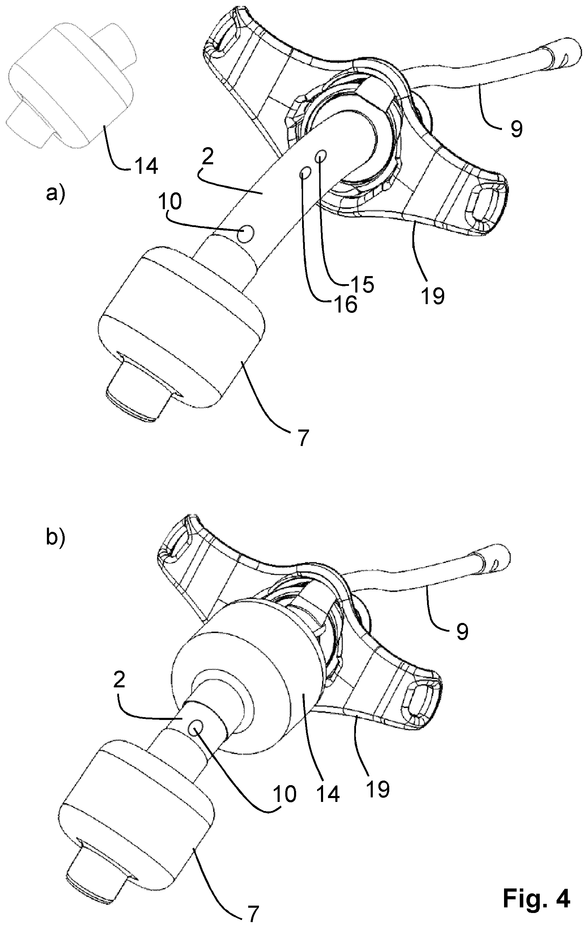

- Figur 4:: Figur 4 zeigt eine perspektivische Ansicht auf eine Ausführungsform einer erfindungsgemäßen Beatmungsvorrichtung, bei der die Sprechluftleitung im Stomaabschnitt und im Trachealabschnitt in Form eines Kanals in der Rohrwand des Kanülenrohres vorgesehen ist, wobei Abbildung a) den Zustand mit nicht montierter Stoma-Manschette darstellt und Abbildung b) den Zustand mit montierter Stoma-Manschette darstellt.

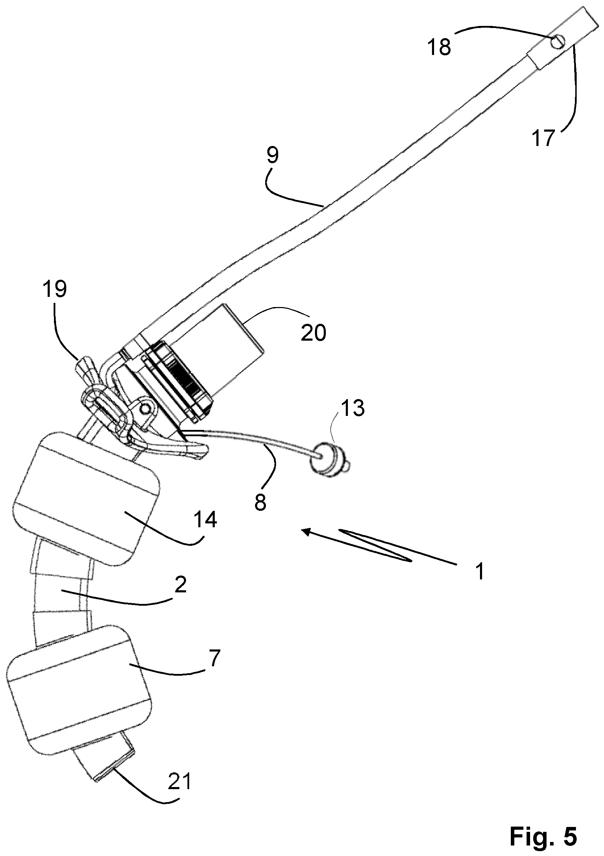

- Figur 5:: Figur 5 zeigt eine Draufsicht von der Seite auf die in Figur 4 b) perspektivisch dargestellte Ausführungsform der erfindungsgemäßen Beatmungsvorrichtung.

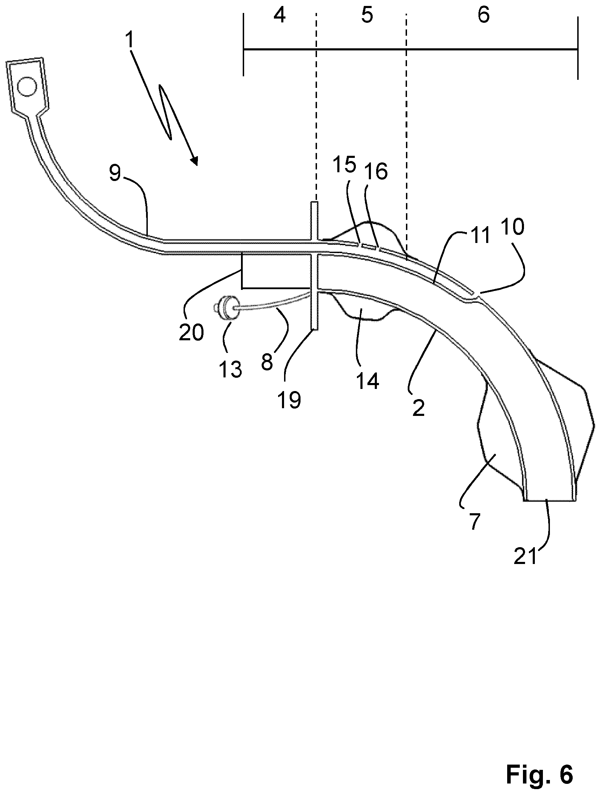

- Figur 6:: Figur 6 zeigt eine schematische Längsschnittansicht einer Ausführungsform einer erfindungsgemäßen Beatmungsvorrichtung, bei der die Sprechluftleitung im Stomaabschnitt und im Trachealabschnitt an der Innenseite der Rohrwand des Kanülenrohrs verläuft.

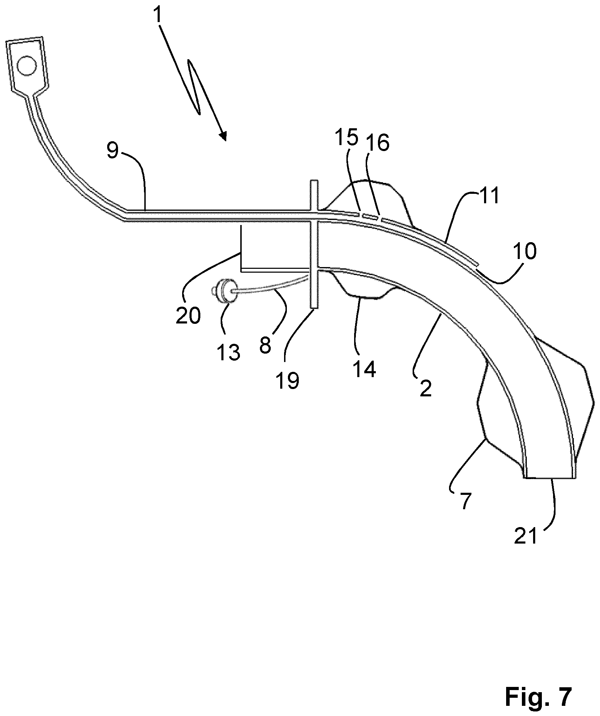

- Figur 7:: Figur 7 zeigt eine schematische Längsschnittansicht einer Ausführungsform einer erfindungsgemäßen Beatmungsvorrichtung, bei der die Sprechluftleitung im Stomaabschnitt und im Trachealabschnitt auf der Außenseite der Rohrwand des Kanülenrohrs verläuft.

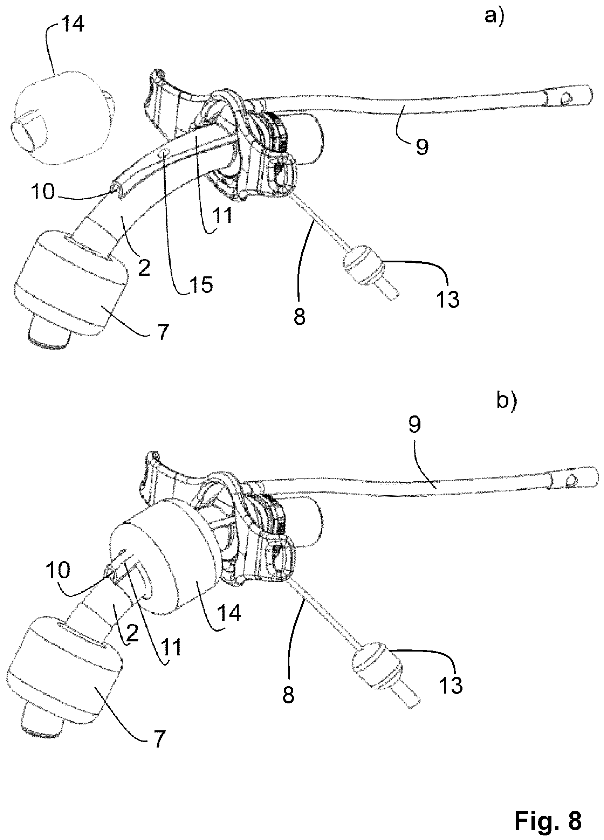

- Figur 8:: Figur 8 zeigt perspektivische Ansichten einer Ausführungsform einer erfindungsgemäßen Beatmungsvorrichtung, bei der die Sprechluftleitung im Stomaabschnitt und im Trachealabschnitt in Form einer auf der Außenwand des Kanülenrohres angebrachten Folienlage vorgesehen ist, wobei Abbildung a) den Zustand ohne montierte Folienmanschette darstellt und Abbildung b) den Zustand mit montierter Folienmanschette darstellt.

Figur 1 zeigt einen schematischen Querschnitt durch den Stomaabschnitt des Kanülenrohres einer Ausführungsform einer erfindungsgemäßen Beatmungsvorrichtung. Die Rohrwand 3 des Kanülenrohres definiert in diesem Bereich einen Kanülenrohrinnenraum, durch den die Atemluft strömen kann. Im oberen Bereich des hier dargestellten Querschnitts ist in der Rohrwand 3 eine Sprechluft-Leitung in Form eines in die Rohrwand 3 eingebetteten Kanals 12 vorgesehen. Durch diesen Kanal 12 kann Sprechluft, die von einer extrakorporal vorgesehenen Sprechluftquelle bereitgestellt wird in distale Richtung geführt werden, um diese in die Trachea einzuleiten. Die Cuffluft-Leitung 8 verläuft bei der hier dargestellten Ausführungsform in der Rohrwand 3. In der Abbildung a) ist ein Querschnitt durch den Kanal 12 dargestellt, in dem dieser geschlossen ist. In der Abbildung b) ist ein Querschnitt durch den Abschnitt des Kanals 12 gezeigt, in dem dieser einen Luftauslass 15 aufweist, der in das Lumen der im Stomaabschnitt sich an der Außenseite der Rohrwand 3 ringförmig um das Kanülenrohr vorgesehenen aufblasbaren Manschette 14 mündet.

Figur 2 zeigt einen schematischen Querschnitt durch den Stomaabschnitt des Kanülenrohres einer weiteren Ausführungsform einer erfindungsgemäßen Beatmungsvorrichtung. Die Rohrwand 3 des Kanülenrohres definiert hier einen kreisrunden Kanülenrohrinnenraum durch den die Atemluft strömen kann. Auf der Außenseite des Kanülenrohres ist auf der Rohrwand 3 die die Sprechluftleitung in Form einer Kunststofflage vorgesehen, die in dem hier dargestellten Zustand ein im Wesentlichen halbmondförmiges Lumen definiert. Die Cuffluft-Leitung 8 verläuft bei der hier dargestellten Ausführungsform in der Rohrwand 3. In Abbildung a) ist der Querschnitt durch einen Abschnitt im Stomabereich gezeigt, in dem die Kunststofflage 11 durchgängig ist. In Abbildung b) ist ein Querschnitt durch einen Bereich gezeigt, indem in der Kunststofflage 11 ein Luftauslass 15 vorgesehen ist, der in das Lumen der im Stomaabschnitt dieser Ausführungsform ringförmig um das Kanülenrohr angeordneten aufblasbaren Manschette 14 mündet.

Figur 3 zeigt einen schematischen Querschnitt durch den Stomaabschnitt einer weiteren Ausführungsform, bei der auf der Innenseite der Rohrwand 3 eine Folienlage 11 vorgesehen ist. Bei dieser Ausführungsform ist in der Rohrwand 3 in einem Bereich des Stomaabschnittes in der Rohrwand 3 ein Luftauslass 15 vorgesehen, der in das Lumen der in diesem Bereich angeordneten ringförmigen aufblasbaren Manschette 14 mündet. Auch hier verläuft die Cuffluft-Leitung 8 in der Rohrwand 3.

In Figur 4 ist eine Ausführungsform der Erfindung mit in der Rohrwand in Form eines Kanals verlaufender Sprechluftleitung dargestellt. Im Stomaabschnitt des Kanülenrohres 2 sind in der Rohrwand zwei Luftauslässe 15, 16 vorgesehen, die in das Lumen einer darüber angeordneten ringförmigen Manschette münden, wobei in der Abbildung a) die Manschette 14 im nicht montierten Zustand separat dargestellt ist, damit die Luftauslässe 15, 16 gut zu erkennen sind. In Abbildung b) ist die Beatmungsvorrichtung dann mit montierter Manschette 14 dargestellt, wodurch die Luftauslässe 15, 16 dann abgedeckt werden. In beiden Abbildungen zu erkennen ist noch der Luftauslass 10, der in der Rohrwand 2 ausgehend von den beiden Luftauslässen 15, 16 in distaler Richtung vorgesehen ist. Über diesen Luftauslass 10 kann die durch die Sprechluftleitung 9 bereitgestellte Sprechluft in die Trachea eingeleitet werden und vom Patienten zur Phonation eingesetzt werden. Zusätzlich zu der im Stomaabschnitt angeordneten Manschette 14 ist hier auch noch die Trachea-Manschette 7 zur Abdichtung der Trachea zu erkennen sowie das Kanülenschild 19, in dem das Kanülenrohr 2 gehalten ist. Die Cuffluft-Leitung ist bei diesen Abbildungen nicht zu erkennen.

In Figur 5 ist die Ausführungsform von Figur 4 noch einmal in der Draufsicht von der Seite dargestellt. Hier ist insbesondere der Übergang des extrakorporalen Bereichs der Sprechluftleitung 9 kurz vor dem Kanülenschild 19 in das Kanülenrohr 2 gut zu erkennen. Gekennzeichnet sind hier außerdem die proximale Atemluftöffnung 20 des Kanülenrohres 2 sowie die distale Atemluftöffnung 21, über welche die Atemluft in das Lumen des Kanülenrohres 2 ein- und ausströmen kann. Die Sprechluftleitung 9 weist an deren proximalen Ende einen Konnektor 17 auf, über den die Verbindung mit einer Druckluftquelle hergestellt werden kann. Durch Abdecken bzw. Freigeben der Ventilöffnung 18 kann der Patient steuern, ob ein kontinuierlich bereitgestellter Druckluftstrom über den extrakorporalen Abschnitt der Sprechluftleitung 9 strömt oder den Weg des geringsten Widerstandes durch die Ventilöffnung 18 nimmt. Bei dieser Abbildung ist auch die Cuffluft-Leitung 8 mit Kontrollballon 13 zu erkennen.

Figur 6 zeigt eine schematische Längsschnittansicht einer Ausführungsform einer erfindungsgemäßen Beatmungsvorrichtung 1, bei der die Sprechluftleitung 9 im Stomaabschnitt 5 und im Trachealabschnitt 6 an der Innenseite der Rohrwand 3 des Kanülenrohres 2 verläuft. Gut zu erkennen sind hier auch die beiden Luftauslässe 15, 16, über die Sprechluft in das Lumen der Manschette 14 einströmen kann, sowie der Luftauslass 10, über welchen die Sprechluft in die Trachea eingeleitet werden kann. Bei dieser Abbildung ist auch die Cuffluft-Leitung 8 mit Kontrollballon 13 zu erkennen.

In Figur 7 ist eine schematische Längsschnittansicht einer Ausführungsform einer erfindungsgemäßen Beatmungsvorrichtung 1 dargestellt, bei der die Sprechluftleitung 9 im Stomaabschnitt und im Trachealabschnitt auf der Außenseite der Rohrwand des Kanülenrohres 2 verläuft. Gut zu erkennen sind auch hier die Luftauslässe 15 und 16 im Stomaabschnitt sowie der Luftauslass 10 im Trachealabschnitt. Auch hier ist die Cuffluft-Leitung 8 mit Kontrollballon 13 zu erkennen.

In Figur 8 ist eine Ausführungsform der erfindungsgemäßen Beatmungsvorrichtung dargestellt, bei der die Sprechluftleitung in Form einer auf der Außenseite des Kanülenrohres 2 aufgebrachten Kunststofflage 11 vorgesehen ist. Die Kunststofflage 11 erstreckt sich bis zum Luftauslass 10 in den Trachealabschnitt. Zusätzlich zum Luftauslass 10 weist die Kunststofflage 11 im Stomaabschnitt einen weiteren Luftauslass 15 auf, der in das Lumen der ringförmigen Manschette 14 mündet, die um das Kanülenrohr 2 angeordnet ist und gleichzeitig auch die Kunststofflage 11 der Sprechluftleitung im Stomaabschnitt umfasst. Strömt Sprechluft durch die Sprechluftleitung 9 und gelangt im Stomaabschnitt in das Lumen, dass sich zwischen der Kunststofflage 11 und der Außenseite der Rohrwand des Kanülenrohres 2 aufspannt, kann ein Teil der Sprechluft über den zusätzlichen Luftauslass 15 im Stomaabschnitt in das Lumen der Manschette 14 gelangen, wodurch die Manschette 14 aufgeblasen wird. Auf diese Weise kann über den Sprechluftvolumenstrom temporär eine Abdichtung des Stomas während des Sprechvorgangs erreicht werden. In der Abbildung b) von Figur 8 ist der Zustand dargestellt, in dem die Manschette 14 um das Kanülenrohr 2 montiert ist. Für Zwecke der Veranschaulichung ist in Abbildung a) die Manschette separat, d.h. im nicht montierten Zustand dargestellt. Hierdurch ist der Luftauslass 15, der bei montierter Manschette 14 in deren Lumen mündet, zu erkennen.

### Bezugszeichen

- 1: Beatmungsvorrichtung
- 2: Kanülenrohr
- 3: Rohrwand
- 4: Extrakorporalabschnitt
- 5: Stomaabschnitt
- 6: Trachealabschnitt
- 7: erste Manschette (Trachea-Manschette)
- 8: erste Luft-Leitung (Cuffluft-Leitung)
- 9: zweite Luft-Leitung (Sprechluft-Leitung)
- 10: Luftauslass
- 11: Lage
- 12: Kanal
- 13: Kontrollballon
- 14: zweite Manschette (Stoma-Manschette)
- 15: Luftauslass
- 16: Luftauslass
- 17: Konnektor
- 18: Ventil
- 19: Kanülenschild
- 20: proximale Atemluftöffnung
- 21: distale Atemluftöffnung

## Patentansprüche

1. Beatmungsvorrichtung (1), insbesondere Tracheostomiekanüle, mit
▪ einem Kanülenrohr (2), dessen Rohrwand (3) einen Extrakorporalabschnitt (4), einen Stomaabschnitt (5) und einen Trachealabschnitt (6) aufweist,
▪ einer ersten aufblasbaren Manschette (7), die sich im Trachealabschnitt (6) an der Außenseite der Rohrwand (3) ringförmig um das Kanülenrohr (2) erstreckt,
▪ einer ersten Luft-Leitung (8), die einen Luftauslass in das Lumen der ersten Manschette (7) aufweist,
▪ einer zweiten aufblasbaren Manschette (14), die sich im Stomaabschnitt (5) an der Außenseite der Rohrwand (3) ringförmig um das Kanülenrohr (2) erstreckt, und
▪ einer zweiten Luft-Leitung (9), die einen Luftauslass (10) in dem Bereich zwischen Stomaabschnitt (5) und der ersten Manschette (7) aufweist,
**dadurch gekennzeichnet, dass** die zweite Luft-Leitung (9) im Bereich des Stomaabschnitts (5) einen zusätzlichen Luftauslass (15) in das Lumen der zweiten Manschette aufweist.

2. Vorrichtung nach Anspruch 1, **dadurch gekennzeichnet, dass** die zweite Luft-Leitung (9) eine reine Sprechluft-Leitung ist, durch die nur Sprechluft geführt wird.

3. Vorrichtung nach Anspruch 1, **dadurch gekennzeichnet, dass** die zweite Luft-Leitung (9) eine subglottische Absaug-Leitung ist, durch die in die eine Richtung subglottisches Sekret abgesaugt werden kann und durch die in die andere Richtung Sprechluft geführt werden kann.

4. Vorrichtung nach einem der vorangehenden Ansprüche, **dadurch gekennzeichnet, dass** die zweite Luft-Leitung (9) in der Rohrwand (3) des Kanülenrohrs (2) verläuft oder auf der Außenseite der Rohrwand (3) des Kanülenrohrs (2) oder an der Innenseite der Rohrwand (3) des Kanülenrohrs (2).

5. Vorrichtung nach einem der vorangehenden Ansprüche, **dadurch gekennzeichnet, dass** die zweite aufblasbare Manschette aus einem thermoplastischen Kunststoffpolymer mit einem Shore A im Bereich von 5 bis 90 besteht.

6. Vorrichtung nach einem der vorangehenden Ansprüche, **dadurch gekennzeichnet, dass** die zweite aufblasbare Manschette aus einem Kunststoffpolymer besteht, das unter Silikon, PVC oder Polyurethan ausgewählt ist.

7. Vorrichtung nach einem der vorangehenden Ansprüche, **dadurch gekennzeichnet, dass** die zweite aufblasbare Manschette bei einem durch die zweite Luftleitung durchgeleiteten Luftstrom von 5 Litern pro Minute einen Außendurchmesser aufweist, der um wenigstens 20% größer ist als der Außendurchmesser des Kanülenrohres auf Höhe der Manschette.

8. Vorrichtung nach einem der vorangehenden Ansprüche, **dadurch gekennzeichnet, dass** die zweite aufblasbare Manschette wenigstens bis zu einem Sprechluft-Druck von 200 mbar reißfest ist.

9. Vorrichtung nach einem der vorangehenden Ansprüche, **dadurch gekennzeichnet, dass** die zweite aufblasbare Manschette von einer Folie mit einer Wandstärke im Bereich von 5 bis 100 µm ausgebildet wird.

10. Vorrichtung nach einem der vorangehenden Ansprüche, **dadurch gekennzeichnet, dass** diese zusätzlich eine separate Absaugleitung (16) zum Absaugen von subglottischem Sekret aufweist.

11. Vorrichtung nach einem der vorangehenden Ansprüche, **dadurch gekennzeichnet, dass** die zweite Luft-Leitung an deren proximalem Ende einen Konnektor (17) für Druckluft, vorzugsweise einen Fingertip-Konnektor, aufweist.

12. Vorrichtung nach einem der vorangehenden Ansprüche, **dadurch gekennzeichnet, dass** die zweite Luft-Leitung im Bereich des Extrakorporalabschnitts aus einem flexiblen Kunststoffschlauch besteht, der eine größere Härte und/oder Wandstärke aufweist, als das Material, aus der die zweite Luft-Leitung im Stomaabschnitt und/oder Trachealabschnitt besteht.

13. Vorrichtung nach einem der vorangehenden Ansprüche, **dadurch gekennzeichnet, dass** die zweite Luft-Leitung am Luftauslass an deren distalem Ende ein Ventil (18) aufweist.

## Claims

1. Ventilation device (1), in particular a tracheostomy cannula, having
▪ a cannula tube (2), of which the tube wall (3) has an extracorporeal portion (4), has a stoma portion (5) and has a tracheal portion (6),
▪ a first inflatable sleeve (7), which extends annularly around the cannula tube (2), on the outer side of the tube wall (3), in the tracheal portion (6),
▪ a first air line (8), which has an air outlet into the lumen of the first sleeve (7),
▪ a second inflatable sleeve (14), which extends annularly around the cannula tube (2), on the outer side of the tube wall (3), in the stoma portion (5), and
▪ a second air line (9), which has an air outlet (10) in the region between stoma portion (5) and first sleeve (7),
**characterized in that** the second air line (9) has in the region of the stoma portion (5) an additional air outlet (15) into the lumen of the second sleeve.

2. Device according to Claim 1, **characterized in that** the second air line (9) is purely a voice-air line, through which only voice air is guided.

3. Device according to Claim 1, **characterized in that** the second air line (9) is a subglottic suction-extraction line, through which subglottic secretions can be extracted by suction in one direction and through which voice air can be guided in the other direction.

4. Device according to one of the preceding claims, **characterized in that** the second air line (9) runs in the tube wall (3) of the cannula tube (2) or on the outer side of the tube wall (3) of the cannula tube (2) or on the inner side of the tube wall (3) of the cannula tube (2) .

5. Device according to one of the preceding claims, **characterized in that** the second inflatable sleeve consists of a thermoplastic polymer with a Shore A in the range from 5 to 90.

6. Device according to one of the preceding claims, **characterized in that** the second inflatable sleeve consists of a plastic polymer which is selected from silicone, PVC and polyurethane.

7. Device according to one of the preceding claims, **characterized in that**, with an air flow passed through the second air line of 5 litres per minute, the second inflatable sleeve has an outer diameter which is at least 20% greater than the outer diameter of the cannula tube at the level of the sleeve.

8. Device according to one of the preceding claims, **characterized in that** the second inflatable sleeve is tear-resistant at least up to a voice-air pressure of 200 mbar.

9. Device according to one of the preceding claims, **characterized in that** the second inflatable sleeve is formed by a film with a wall thickness in the range from 5 to 100 µm.

10. Device according to one of the preceding claims, **characterized in that** said device additionally has a separate suction-extraction line (16) for extracting subglottic secretions by suction.

11. Device according to one of the preceding claims, **characterized in that** the second air line has at the proximal end thereof a connector (17) for compressed air, preferably a fingertip connector.

12. Device according to one of the preceding claims, **characterized in that**, in the region of the extracorporeal portion, the second air line consists of a flexible plastic hose which has a greater hardness and/or wall thickness than the material of which the second air line consists in the stoma portion and/or tracheal portion.

13. Device according to one of the preceding claims, **characterized in that** the second air line has a valve (18) at the air outlet on the distal end thereof.

## Revendications

1. Dispositif de ventilation (1), notamment canule de trachéotomie, avec
▪ un tube de canule (2), dont la paroi de tube (3) présente une section extracorporelle (4), une section stomatique (5) et une section trachéale (6),
▪ un premier manchon gonflable (7) qui s'étend de manière annulaire autour du tube de canule (2) dans la section trachéale (6) sur le côté extérieur de la paroi de tube (3),
▪ un premier conduit d'air (8), qui présente une sortie d'air dans la lumière du premier manchon (7),
▪ un deuxième manchon gonflable (14) qui s'étend de manière annulaire autour du tube de canule (2) dans la section stomatique (5) sur le côté extérieur de la paroi de tube (3), et
▪ un deuxième conduit d'air (9), qui présente une sortie d'air (10) dans la zone entre la section stomatique (5) et le premier manchon (7),
**caractérisé en ce que** le deuxième conduit d'air (9) présente, dans la zone de la section stomatique (5), une sortie d'air supplémentaire (15) dans la lumière du deuxième manchon.

2. Dispositif selon la revendication 1, **caractérisé en ce que** le deuxième conduit d'air (9) est un conduit d'air de parole uniquement, à travers lequel seul de l'air de parole est acheminé.

3. Dispositif selon la revendication 1, **caractérisé en ce que** le deuxième conduit d'air (9) est un conduit d'aspiration sous-glottique à travers lequel des sécrétions sous-glottiques peuvent être aspirées dans une direction et à travers lequel de l'air de parole peut être amené dans l'autre direction.

4. Dispositif selon l'une quelconque des revendications précédentes, **caractérisé en ce que** le deuxième conduit d'air (9) s'étend dans la paroi de tube (3) du tube de canule (2) ou sur le côté extérieur de la paroi de tube (3) du tube de canule (2) ou sur le côté intérieur de la paroi de tube (3) du tube de canule (2).

5. Dispositif selon l'une quelconque des revendications précédentes, **caractérisé en ce que** le deuxième manchon gonflable est constitué d'un polymère thermoplastique ayant une dureté Shore A dans la plage allant de 5 à 90.

6. Dispositif selon l'une quelconque des revendications précédentes, **caractérisé en ce que** le deuxième manchon gonflable est constitué d'un polymère plastique choisi parmi la silicone, le PVC ou le polyuréthane.

7. Dispositif selon l'une quelconque des revendications précédentes, **caractérisé en ce que**, pour un débit d'air de 5 litres par minute passant par le deuxième conduit d'air, le deuxième manchon gonflable présente un diamètre extérieur supérieur d'au moins 20 % au diamètre extérieur du tube de canule à hauteur du manchon.

8. Dispositif selon l'une quelconque des revendications précédentes, **caractérisé en ce que** le deuxième manchon gonflable est résistant à la déchirure au moins jusqu'à une pression d'air de parole de 200 mbar.

9. Dispositif selon l'une quelconque des revendications précédentes, **caractérisé en ce que** le deuxième manchon gonflable est formé par un film ayant une épaisseur de paroi dans la plage allant de 5 à 100 µm.

10. Dispositif selon l'une quelconque des revendications précédentes, **caractérisé en ce qu'**il présente en outre un conduit d'aspiration séparé (16) pour aspirer les sécrétions sous-glottiques.

11. Dispositif selon l'une quelconque des revendications précédentes, **caractérisé en ce que** le deuxième conduit d'air présente à son extrémité proximale un connecteur (17) pour l'air comprimé, de préférence un connecteur Fingertip.

12. Dispositif selon l'une quelconque des revendications précédentes, **caractérisé en ce que** le deuxième conduit d'air dans la zone de la section extracorporelle est constitué d'un tuyau en plastique flexible qui présente une dureté et/ou une épaisseur de paroi supérieure au matériau dont est constitué le deuxième conduit d'air dans la section stomatique et/ou la section trachéale.

13. Dispositif selon l'une quelconque des revendications précédentes, **caractérisé en ce que** le deuxième conduit d'air présente une vanne (18) à la sortie d'air à son extrémité distale.
